(19) Europäisches Patentamt
European Patent Office
Office européen des brevets

(11) Publication number: 0 291 626 A2

(12) # EUROPEAN PATENT APPLICATION

(21) Application number: 88100324.8

(51) Int. Cl.[4]: C07C 121/75 , C07C 120/00

(22) Date of filing: 18.11.83

(30) Priority: 22.11.82 US 443513
22.11.82 US 443763
22.11.82 US 443764

(43) Date of publication of application:
23.11.88 Bulletin 88/47

(60) Publication number of the earlier application in accordance with Art.76 EPC: 0 109 681

(84) Designated Contracting States:
AT BE CH DE FR GB IT LI LU NL SE

(71) Applicant: E.I. DU PONT DE NEMOURS AND COMPANY
1007 Market Street
Wilmington Delaware 19898(US)

(72) Inventor: Stoutamire, Donald W.
904 Bel Passi Dr.
Modesto Calif. 95350(US)
Inventor: Tieman, Charles H.
2209 Fremont St.
Modesto Calif. 95350(US)
Inventor: Dong, Walter
14428 Rive Forest
Houston Texas 77079(US)

(74) Representative: Kraus, Walter, Dr. et al
Patentanwälte Kraus, Weisert & Partner
Thomas-Wimmer-Ring 15
D-8000 München 22(DE)

(54) **Process for the preparation of optically-active cyanomethyl esters.**

(57) Stereoisomerically-enriched cyanomethyl esters are prepared by treating a non-symmetrical ketene or an alpha-chiral carboxylic acid halide or reactive derivative thereof with an optically-active alpha-hydroxynitrile. Certain optically-active optionally substituted S-alpha-cyano-3-phenoxybenzyl alcohol intermediates are prepared by treating the corresponding aldehyde or ketone with a source of hydrogen cyanide in the presence of a substantially water-immiscible, aprotic solvent and a cyclo(D-phenylalanyl-D-histidine) as a catalyst.

EP 0 291 626 A2

# PROCESS FOR THE PREPARATION OF OPTICALLY-ACTIVE CYANOMETHYL ESTERS

## Background of the Invention

### Field of the Invention

The present invention is directed to processes for the preparation of cyanomethyl esters having chiral centers in both the acid and alcohol moieties, novel catalysts for the production therefor, and preparation of certain optically-active, optionally substituted S-alpha-cyano-3-phenoxybenzyl alcohol intermediates to said esters.

### Description of the Prior Art

Stereoisomers of chiral-cyanomethyl esters of alpha-chiral carboxylic acids or the acids themselves usually have different effects in biological systems. In the past, it usually had not been simple to prepare such optically-active cyanomethyl esters directly because the corresponding chiral alpha-hydroxynitriles were not always readily available or synthesis methods gave products with low or no enrichment. Even when these optically-active alpha-hydroxynitriles were available or became more readily available, the optically-active acids were not always readily accessible. Often, the optically-active acids were obtained by classical resolution, which was usually time consuming and not practical on a large scale. Optically-active alpha-hydroxybenzeneacetonitriles are known in the art and are of interest, per se, and as intermediates, e.g. to esters. In alcohol derived pyrethroid esters, those having an (alpha-S)-alpha-hydroxynitrile moiety coupled with the appropriate pyrethroid acid usually have the highest pesticidal activity. However, such (alpha-S)-alpha-hydroxynitriles have not been easily obtained in the past because they were usually prepared by resolution.

The present process provides a process for preparing chiralcyanomethyl esters of alpha-chiral-carboxylic acids in high yield by a direct synthesis method, avoiding the cumbersome classical resolution of the corresponding optically-active acids and alcohols.

## Summary of the Invention

The present invention is directed to a process for the preparation of an optically-active cyanomethyl ester of an alpha-chiral(optically-active) carboxylic acid (i.e. alpha-chiral-cyanomethyl esters of alpha-chiral carboxylic acids), or a mixture enriched therein, which comprises treating a non-symmetrical ketene with a racemic or an optically-active alpha-hydroxynitrile. The optically-active cyanomethyl ester products include those of formula I below

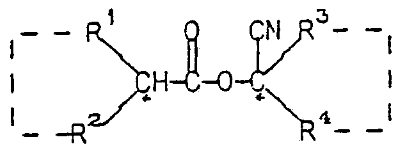

I

wherein $R^1$, $R^2$, $R^3$ and $R^4$ are substituents; * denotes an asymetrically substituted carbon atom, and the broken lines are optional bonds.

The reaction is conducted in the presence or absence of a solvent. When a solvent is used the solvent is preferably a non-hydroxylic solvent such as hydrocarbons, chlorinated hydrocarbons, ethers and the like. For example, suitable solvents are alkanes containing from 5 to 10 carbon atoms such as n-pentane, n-hexane, n-heptane, n-octane, n-nonane, n-decane and their isomers. Petroleum fractions rich in alkanes are also suitable, for example, gasoline with a boiling range at atmospheric pressure of between 40° and 65° C, between 60° and 80° C or between 80° and 110° C. Petroleum ether is also suitable. Cyclohexane and methylcyclohexanes are examples of useful cycloalkanes containing from 6 to 8 carbon atoms. Aromatic

hydrocarbon solvents can contain from 6 to 10 carbon atoms, for example, benzene, toluene, o-, m- and p-xylene, the trimethylbenzenes, p-ethyltoluene and the like. Suitable chlorinated hydrocarbons contain from 1 to 4 chlorine atoms in combination with an alkane chain contianing from 1 to 4 carbon atoms or with a benzene ring, for example, carbon tetrachloride, chloroform, dichloromethane, 1,2-dichloroethane, trichloroethane, perchloroethane, chlorobenzene and 1,2- or 1,3-dichlorobenzene and the like. Ethers are generally those containing from 4 to 6 carbon atoms such as diethyl ether, methyl tert-butyl ether and diisopropyl ether and the like. The solvent is preferably an aromatic solvent, especially toluene.

Any non-symmetrical ketene is useful (provided it does not contain substituent groups which form other stable reaction products with the alpha-hydroxynitrile). The non-symmetrical ketenes have the formula II

$$R^2\text{-}\overset{\displaystyle R^1}{\overset{|}{C}} = C = O \qquad \text{II}$$

wherein $R^1$ and $R^2$ each independently is a different alkyl, aralkyl, alkoxy, aryloxy, alkylthio, alkylsulfonyl, arylthio, or arylsulfonyl group containing from 1 to 10 carbon atoms, or a cycloalkyl group containing 3 to 7 ring carbon atoms, or $R^2$ is also an alkenyl or alkynyl group containing 2 to 10 carbon atoms; a naphthyl group; a phenyl group; a heterocyclic group containing 5 to 6 ring atoms, one of which is oxygen, sulfur or nitrogen, and the remainder are carbon atoms; or an amino group disubstituted by acyl or alkyl containing up to 10 carbon atoms or a phenyl group; or $R^1$ and $R^2$, when taken together with the carbon atom to which they are attached, form a non-symmetrical cycloalkyl group containing 4 to 7 ring carbon atoms and 4 to 14 carbon atoms. The $R^1$ and $R^2$ groups can be optionally substituted by one or more of halogen atoms having an atomic number of from 9 to 35, alkyl or haloalkyl containing 1 to 4 carbon atoms, alkenyl or haloalkenyl containing 2 to 4 carbon atoms, haloalkoxy or alkoxy of 1 to 4 carbon atoms, haloalkylthio or alkylthio of 1 to 4 carbon atoms or equivalent kinds and sizes of substituents which may contain the same or greater carbon number.

One embodiment of non-symmetrical ketenes used in the process of the invention is that which results in pyrethroid esters, including those estrs having an acid moiety described in U.S. patents 4,062,968, 4,137,324 and 4,199,595. Examples of such ketenes include those having the formula II in which $R^1$ is isopropyl or cyclopropyl; $R^2$ is an alkyl group containing 1 to 6 carbon atoms; an alkenyl group containing 2 to 6 carbon atoms; a naphthyl group; a phenyl group or a (benzyloxycarbonyl)phenylamino group, each optionally ring-substituted by one or more of halogen, alkyl, haloalkyl, alkoxy, haloalkoxy in which the halogens are bromine, chlorine or fluorine, and the alkyl groups contain 1 to 4 carbon atoms.

Of particular interest as non-symmetrical ketene reactants because their resulting esters are usually highly pesticidally active are those ketenes having the formula II in which $R^1$ is isopropyl; $R^2$ is a phenyl group para-substituted by halogen, alkyl or haloalkoxy, in which the halogen is, e.g. chlorine or fluorine and the alkyl contains 1 to 4 carbon atoms, e.g. methyl.

For example, the non-symmetrical ketene is (4-chlorophenyl)isopropylketene, (4-(difluoromethoxy)-phenyl)isopropylketene, ((4-(trifluoromethyl)-3-chlorophenyl)(benzyloxycarbonyl)amino)isopropylketene, and the like.

Any racemic or optically-active alpha-hydroxynitrile is useful (provided it does not contain substituent groups which form other stable reaction products with the non-symmetrical ketene or the catalyst, when present). Preferably, the alpha-hydroxynitrile is a symmetrical or non-symmetrical, racemic or optically-active alpha-hydroxynitrile of formula III

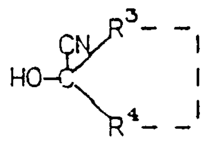

III

wherein $R^3$ is an optionally-substituted hydrocarbyl or heterocyclic group; and $R^4$ is an optionally substituted hydrocarbyl group or a hydrogen atom or $R^3$ and $R^4$ together with the carbon atom to which they are attached form a carbocyclic group as denoted by the dotted line.

The hydrocarbyl groups represented by $R^3$ and $R^4$ in the formula III may be, for example, an alkyl, a cycloalkyl or an aryl group of up to 20 carbon atoms, preferably up to 10 carbon atoms, or $R^3$ in the formula III may be a carbocyclic or an O or S heterocyclic aryl group. Examples of carbocyclic aryl groups are phenyl, 1-naphthal, 2-naphthyl and 2-anthryl groups. Heterocyclic aromatic groups are derived from heteroaromatic compounds which are defined as in Kirk-Othermer, "Encyclopedia of Chemical Technology", Second Edition, Volume 2 (1963), page 702: obtained by replacement of one or more carbon atoms of a carbocyclic aromatic compound by a heteroatom selected from O or S and also include those heterocyclic

compounds having five-membered rings which show aromatic characteristics and are mentioned on page 703 of said volume. Optional substituents include one or more of halogen atoms having an atomic number of from 9 to 35, inclusive, or an alkyl, alkenyl or alkoxy group containing 1 to 6 carbon atoms, each optionally substituted by one or more halogen atoms, optionally substituted phenoxy, phenyl, benzyl or benzoyl and equivalent kinds of substituents. Illustrative examples of the optically-active alpha-hydroxynitriles include alpha-hydroxy-alpha-methylbutyronitrile, alpha-hydroxy-alpha-methylbenzeneacetonitrile, alpha-hydroxyisobutyronitrile, and the like.

Preferably, the alpha-hydroxynitrile compound has the formula

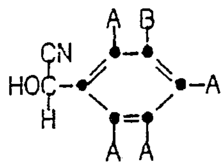

wherein each A is independently a hydrogen atom, a halogen atom having an atomic number of from 9 to 35, inclusive, or an alkyl, alkenyl or alkoxy group containing 1 to 6 carbon atoms, each optionally substituted by one or more halogen atoms having an atomic number of from 9 to 35, inclusive; B is a hydrogen atom, a halogen atom having an atomic number of from 9 to 35; inclusive, or an alkyl; alkenyl or alkoxy group containing 1 to 6 carbon atoms, each optionally substituted by one or more halogen atoms having an atomic number of from 9 to 35, inclusive, or is a group

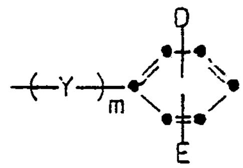

in which Y is O, $CH_2$, or C(O); m is 0 or 1 and D and E each independently is a hydrogen atom, a halogen atom having an atomic number of from 9 to 35, inclusive, or an alkyl, alkenyl or alkoxy group contaiing 1 to 6 carbon atoms, each optionally substituted by one or more halogen atoms having an atomic number of from 9 to 35, inclusive.

Preferably, the alpha-hydroxynitrile can have the R- or S-configuration, and therefore, include either the R- or, preferably S-alpha-hydroxynitrile of the formula

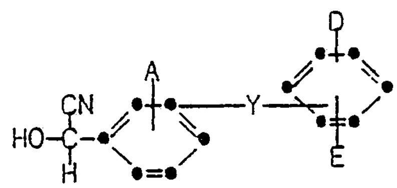

wherein Y is O, $CH_2$, or C(O); each A, D and E independently is a hydrogen atom, a halogen atom having an atomic number of from 9 to 35, inclusive, or an alkyl, alkenyl or alkoxy group containing 1 to 6 carbon atoms, each optionally substituted by one or more halogen atoms having an atomic number of from 9 to 35, inclusive. Preferably, Y is O. Preferably, each A, D or E independently is a hydrogen atom, a fluorine atom, a chlorine atom, a methyl group, a trifluoromethyl group or a methoxy group. Preferably, one of D and E is a hydrogen atom. An especially preferred subclass of S-alpha-hydroxynitriles are those of the formula above in which D is a hydrogen atom and A and E each independently is a fluorine atom or a hydrogen atom, and, preferably, when either A or E is fluorine, each is located at the 4-position of the ring relative to the benzyl carbon when A or relative to the Y=O bearing carbon atom when E. Especially suitable alcohols are when A is a fluorine atom at the 4-position and E is a hydrogen atom.

Examples of alpha-hydroxynitriles of the above formula include S-alpha-cyano-3-phenoxybenzyl alcohol, S-alpha-cyano-4-fluoro-3-phenoxybenzyl alcohol, S-alpha-cyano-3-(4-fluorophenoxy)benzyl alcohol, and their corresponding enantiomers and the like.

The present invention is directed to a process for the preparation of a sterioisomerically-enriched

cyanomethyl ester by treating a non-symmetrical ketene with a racemic or optically-active alpha-hydroxynitrile (in which both the hydroxy and nitrile substituents are attached to the same carbon atom) in the absence of a catalyst or in the presence of an achiral tertiary amine catalyst or of an optically-active (chiral) tertiary-amine catalyst.

The optically-active (chiral) tertiary-amine catalyst is any optionally-substituted alkyl, cycloalkyl, aromatic or heterocyclic mono-or polyamine containing up to 40 carbon atoms (including polymers and copolymers and amine salts and the like), which will not interfere with the reaction. The amine is preferably a moderate to weakly basic amine. The optically-active amines, polymers and copolymers are conventional kinds of materials known in the art and can be prepared by known methods except for certain novel ketene reaction products discussed below. For example, numerous optically-active amines are specifically disclosed in Newman, P., "Optical Resolution Procedures for Chemical Compounds", Vol. 1, Amines and Related Compounds, Optical Resolution Information Center, Manhattan College, Riverdale, N.Y., Library of Congress Catalog Card No. 78-61452. This reference also discloses optically-active mono-, di- and polyamines which can be polymerized and co-polymerized by known procedures to form optically-active polymeric amines for use in the invention.

One embodiment of the optically-active amine catalyst comprises a substituted optically-active amino acid which is preferably any acyclic, carbocyclic, aromatic or heterocyclic amino acid containing up to 20 carbon atoms, preferably up to 10 carbon atoms, additionally substituted by a moderate to weakly basic nitrogen base substituent or is the reaction product thereof with about one to about three moles of a ketene. Suitable nitrogen-base substituents include optionally substituted nitrogen-heterocyclic groups or amino groups, each optionally substituted by alkyl or cycloalkyl groups containing 1 to 6 carbon atoms or by optionally substituted phenyls. Other optional substituents include hydroxy, alkyl, alkoxy, amino, alkylthio, phosphoryloxy, amido and the like. Examples of nitrogen-heterocyclic groups include thiazolyl, imidazolyl, pyrrolyl, benzopyrrolyl and the like.

Non-limiting examples of the optically-active catalyst include beta-aminoalanine, ornithine, canavanine, anserine, kynurenin, mimosine, cystathionine, ephedrin, acylated ephedrins, histidinol, citrulline, carbamoylserine, cinchonine, quinine or acylated quinuclidinyl alcohols.

Another embodiment of the amine catalysts are the heterocyclic amines and polymers of heterocyclic amines. Non-limiting examples include di- and polyaziridines, polymers of acryloylcinchonines alone or with N,N-diacryloylhexamethylenediamine, di- and poly(iminoisobutylethylene), polymers of (N-benzyl-2-pyrrolidinylmethyl ester) with acrylate or a lower alkanoic acid, and like materials.

In another embodiment of the invention, the catalyst is an optically-active histidine-containing peptide; or is a histidine-containing di- or polypeptide; in which at least one of the histidinyl free N-H and free COOH groups is optionally modified with a protecting group into the form of an amide (or acid addition salt thereof) and an ester group, respectively; or the reaction product of one mole of a histidine or a histidine-containing di- or polypeptide with about one mole of a ketene per mole of histidine group.

The di- or polypeptide is linear or cyclic. These peptides usually contain from about 2 up to about 16 peptide units, preferably 2 to 4 peptide units. Nitrogen-substituted amino acids, including these histidine-containing di- and polypeptides, are prepared by conventional peptide synthesis, for example, as in Greenstein, J. P. and M. Winitz, "Chemistry of the Amino Acids", John Wiley & Sons, Inc., New York, 1961.

The dipeptides of the histidine-containing catalyst are preferred, especially in the cyclic dipeptide form. The di- or polypeptides may also contain an alanine, and those prepared with alanine, phenylalanine, or alanine derivatives, are preferred.

In one embodiment of the invention, the asymmetric carbon atoms in the histidine-containing peptide is a catalyst in the D configuration, although the L-configuration in the histidine-containing peptide is also useful. Choice of chirality in the catalyst can be made so as to provide the chirality desired in the product.

Functional groups in the amino acid catalyst can contain protecting groups; any conventional amino acid protecting group known in the art can be used. For exampale, the protecting group is an organic acid in the case of the free N-H or an alcohol in the case of the free COOH. Any organic acid and alcohol which will not interfere with the reaction can be used as the protecting group. Preferably, the protecting group is another amino acid. Any amino acid can be used, but, preferably, the amino acid is non-heterocyclic and is a monoamino or diamino-alkanoic or aralkanoic acid, such as alanine, phenylalanine, glutamic acid, glycine and the like.

Acid addition salts of the amine catalysts are formed with any acid that will not interfere with the reaction. Suitable inorganic acids include hydrohalogenic acids, such as hydrochloric or hydrobromic; sulfur acids, such as sulfuric or toluenesulfonic; and phosphorus acids, such as phosphoric or phenylphosphonic; and organic acids, such as oxalic acid and the like, are also suitable to form the salts.

When preparing the di- or polypeptide catalyst also having an alanine (containing moiety), it is prepared

from alanine or its derivatives; this includes alanine, beta-aminoalanine, beta-phenylalanine, 3,4-dihydroxyphenylalanine and the like. When preparing the catalyst from a histidine (containing moiety), it is preferably histidine, 3-methylhistidine, 1-methylhistidine, 1-ethyl-histidine, 1-propyl-histidine or 1-benzylhistidine and the like. Preferably, the catalyst is a cyclic dipeptide containing a histidine moiety and an alanine moiety.

The peptide adducts (reaction products) with ketene are novel and comprise another aspect of the invention. They are prepared to contain from about one mole to three moles of a ketene per mole of peptide and, preferably, about two, and especially, about one mole of a ketene per mole of a (cyclic) dipeptide. Obviously, it is preferable to form the adduct in situ with the non-symmetrical ketene reactant of the process, which is described below under process conditions. However, treatment of the optically-active catalyst with about 1 to 3 moles of a ketene, preferably in the absence of a solvent or in the presence of any solvent used in preparing the ketene, is suitable. The ketene may also be a similar kind but symmetrical ketene, e.g. dimethylketene, diphenylketene, etc., or ketene itself.

Non-limiting examples of the optically-active histidine-containing catalyst include histidine, alpha-methylhistidine, 1-methylhistidine, 3-methylhistidine, cyclo(histidyl-histidine), (benzyloxy-carbonylalanyl)-histidine methyl ester, cyclo(alanyl-histidine), cyclo(beta-phenylalanyl-histidine), histidine methyl ester hydrochloride, histidine ethyl ester dihydrochloride, anserine, cyclo(valyl-histidine), tidine), glycyl-histidine, cyclo(phenylalanyl-glycyl-histidine), cyclo(leucyl-histidine), cyclo(homophenylalanyl-histidine), cyclo-(phenylalanyl-methylhistidine), N-alpha-(beta-naphthoyl)histidine, histidyl-alanine, histidyl-phenylalanamide hydrochloride, histidyl-phenylalanine, cyclo(histidyl-proline), cyclo(glycyl-histidine) in free or protected form or a reaction product of these materials with a ketene, especially (4-chlorophenyl)isopropylketene. Also, cyclo(beta-phenylalanyl-histidine) adduct with (4-(difluoromethoxy)phenyl)isopropylketene, histidine adduct with dimethylketene, cyclo(glycyl-histidine) adduct with (4-(difluoromethoxy)phenyl)isopropylketene or histidyl-alanine adduct with dimethylketene and the like.

In one subclass of the invention, the peptide catalyst has the formula

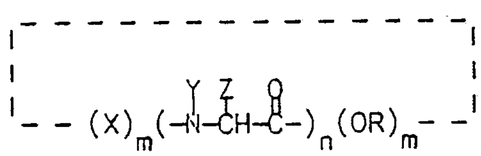

wherein X is H, alkyl or $R-\overset{O}{\overset{\|}{C}}$ , each R is independently alkyl or cycloalkyl of up to 7 carbon atoms, optionally substituted phenyl, benzyl or the like, each of the n units of $(-\overset{Y}{N}-\overset{Z}{C}H-\overset{O}{\overset{\|}{C}}-)$ is independently substituted in which Y is hydrogen, acyl, alkyl, or aralkyl of up to 10 carbon atoms; Z is the residue of common amino acids that do not interfere in the process of the invention including benzyl, 3-carboxypropyl, 3-aminopropyl, mercaptomethyl, 4-hydroxybenzyl, imidazol-4-ylmethyl; each m is 0 or 1, n is 2 to 16; when each m is 0, the catalyst has a cyclic structure denoted by the dotted line; with the proviso that at least one histidine or substituted histidine unit is included in the catalyst; or reaction products of the above catalysts with one to three moles of ketene.

When the peptide catalysts are prepared by conventional methods in the presence of solvent (e.g. water), they can, if solid, also contain solvent (e.g. water) of crystallization. The optically-active, nitrogen-based amino acid, e.g., histidine-containing peptide, catalyst of the invention, thus, includes the presence or absence of solvent (e.g. water) of crystallization when solid.

The achiral tertiary amine catalyst is any optionally-substituted alkyl, cycloalkyl, aromatic or heterocyclic tertiary amine containing up to about 40 carbon atoms (including polymers and copolymers and amine salts), which will not interfere with the reaction. The amine is a moderately to strongly basic amine. The achiral tertiary amines, polymers and copolymers are conventional kinds of materials known in the art and can be prepared by known methods.

Illustrative examples of the achiral tertiary amine catalyst include 1-methylimidazole, pyridine, 2,6-lutidine, triethylamine, trimethylamine, N,N-dimethylaniline, Diisopropylethylamine, 1,4-diazabicyclo[2.2.2]-octane, and the like.

In one embodiment of the invention, the achiral tertiary amine catalyst is an acyclic, carbocyclic, aromatic or heterocyclic amine containing up to about 20 carbon atoms. Preferably, the tertiary amine is an acyclic or aromatic amine contianing from 3 to 8 carbon atoms and conveniently an acyclic tertiary amine, such as triethylamine or trimethylamine and the like.

The term achiral tertiary amine herein includes acid addition salts thereof. The acid addition salts are

formed with any acid which will not interfere with the reaction. Suitable inorganic acids include hydrohalogenic acids, such as hydrochloric or hydrobromic; sulfur acids, such as sulfuric or sulfonic; and phosphorus acids, such as phosphoric or phosphonic; and organic acids, such as oxalic acid and the like, are also suitable to form the salts.

Alternatively, the process of the invention comprises treating the non-symmetrical ketene as previously defined with an aldehyde or ketone and a source of cyanide ions, optionally in the presence of the achiral or chiral (optically-active) catalyst previously defined. The reaction is preferably conducted in a nonhydroxylic solvent of the type previously described.

Any aldehyde or ketone (carbonyl compound) is useful (provided it does not contain substituent groups that form other stable reaction products with cyanide ions or with the catalyst). Preferably, the aldehyde or ketone has the formula

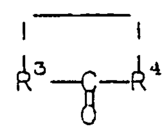

IV

wherein $R^3$ is an optionally substituted hydrocarbyl or heterocyclic group and $R^4$ is an optionally substituted hydrocarbyl group or a hydrogen atom, or, alternatively, $R^3$ and $R^4$ together with the carbon atom to which they are attached form a carbocyclic group.

The hydrocarbyl groups represented by $R^3$ and $R^4$ in the formula IV may be, for example, an alkyl, a cycloalkyl or an aryl group of up to 20 carbon atoms, preferably up to 10 carbon atoms, or $R^3$ in the formula IV may be a carbocyclic or an O or S heterocyclic aryl group. Examples of carbocyclic aryl groups are phenyl, 1-naphthyl, 2-naphthyl and 2-anthryl groups. Heterocyclic aromatic groups are derived from heteroaromatic compounds which are defined as in Kirk-Othmer, "Encyclopedia of Chemical Technology", Second Edition, Volume 2 (1963), page 702: obtained by replacement of one or more carbon atoms of a carbocyclic aromatic compound by a heteroatom selected from O or S -- and also include those heterocyclic compounds having five-membered rings which show aromatic characteristics and are mentioned on page 703 of said volume. Such aldehydes and ketone compounds are described in U.S. patent 4,132,728. Optional substituents include one or more of halogen atoms having an atomic number of from 9 to 35, inclusive, or an alkyl, alkenyl or alkoxy group containing 1 to 6 carbon atoms, each optionally substituted by one or more halogen atoms, or optionally substituted phenoxy, phenyl, benzyl or benzoyl and equivalent kinds of substituents.

Preferably, an aromatic aldehyde is used of the formula

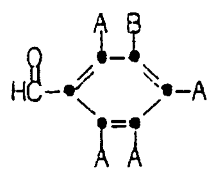

wherein each A is independently a hydrogen atom, a halogen atom having an atomic number of from 9 to 35, inclusive, or an alkyl, alkenyl or alkoxy group containing 1 to 6 carbon atoms, each optionally substituted by one or more halogen atoms having an atomic number of from 9 to 35, inclusive; B is a hydrogen atom, a halogen atom having an atomic number of from 9 to 35, inclusive, or an alky, alkenyl or alkoxy group containing 1 to 6 carbon atoms, each optionally substituted by one or more halogen atoms having an atomic number of from 9 to 35, inclusive; or is a group

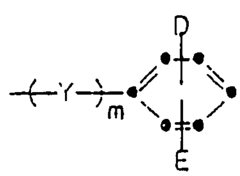

in which Y is O, $CH_2$, or C(O); m is 0 or 1 and D and E each independently is a hydrogen atom, a halogen atom having an atomic number of from 9 to 35, inclusive, or an alkyl, alkenyl or alkoxy group containing 1

to 6 carbon atoms, each optionally substituted by one or more halogen atoms having an atomic number of from 9 to 35, inclusive.

Preferably, an aldehyde is used corresponding to the alpha-hydroxynitrile previously defined and, thus, has the formula

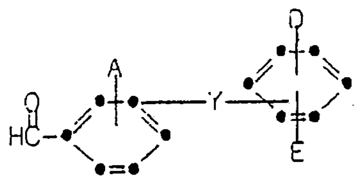

wherein A, D, E and Y have the same meanings as given in the formula above.

Examples of suitable aldehydes of the formula above include 3-phenoxybenzaldehyde, 4-fluoro-3-phenoxybenzaldehyde and the like.

The source of cyanide ions is hydrogen cyanide or agent which generates hydrogen cyanide, such as an alpha-hydroxynitrile such as acetone cyanohydrin, under the reaction conditions. The molar ratio of hydrogen cyanide to one mole of aldehyde or ketone is from about 1.0 to about 3.0 and, preferably, from about 1.1 to about 2.0.

The preparation of the cyanomethyl esters is conducted by adding the non-symmetrical ketene to the alpha-hydroxynitrile, or to the aldehyde or ketone and a source of cyanide ions, preferably dissolved in a solvent containing the catalyst, agitating the mixture, e.g., by stirring, and maintaining the reaction conditions for an amount of time to effect the formation of the optically-active ester. Separation and recovery of the optically-active ester product are achieved by conventional techniques, including extraction and the like.

The amount of catalyst can vary. For example, depending on the tertiary achiral amine used, it can readily vary in the range of from about 1 to about 100 percent based upon the weight of the alpha-hydroxynitrile, aldehyde or ketone present, preferably about 1 to about 10 mole percent. When the catalyst is a chiral tertiary amine, it can be used in the range of from about 0.1 to about 10 mole percent and, preferably, 0.1 to about 5 mole percent based upon the weight of the alpha-hydroxynitrile, aldehyde or ketone present, especially about 1.5 to about 5 mole percent.

The molar ratio of the starting materials, non-symmetrical ketene and alpha-hydroxynitrile, aldehyde or ketone can vary. For example, the molar ratio of ketene to alpha-hydroxynitrile is from about 5:1 to about 1:5 and, preferably, from about 2:1 to about 1:2. However, it is desirable to have a molar excess of ketene to alpha-hydroxynitrile, aldehyde or ketone of from about 1:1.1 to about 1:1.5.

The temperature of the reaction for the preparation of cyanomethyl esters as well as the pressure can vary. At normal pressures, the temperature is from about -10°C to about 50°C, more or less. Ambient temperatures of about 0°C to about 35°C are convenient and especially from about 0° to about 15°C.

The alpha-hydroxynitriles and their corresponding aldehydes or ketones are generally known in the literature. They can be either directly synthesized chemically or often enzymatically or, in the case of optically-active alpha-hydroxynitrile, resolves by methods conventionally known in the art, including those described in U.S. patents 3,649,457 and 4,273,727, Oku et al., J.C.S. Chem. Comm., pages 229-230 (1981) and Becker et al., J. Amer. Chem. Soc., 88, pages 4299-4300 (1966) and the like.

The optically-active, optionally substituted S-alpha-cyano-3-phenoxybenzyl alcohols are also prepared by treating the corresponding aldehyde or ketone with hydrogen cyanide in a substantially water-immiscible, aprotic solvent and in the presence of a cyclo(D-phenylalanyl-D-histidine) dipeptide catalyst. Such preparation of optionally substituted S-alpha-cyano-3-phenoxybenzyl alcohols is disclosed and claimed herein.

The catalyst is prepared by conventional peptide synthesis, for example, as in Greenstein, J. P. and M. Winitz, "Chemistry of the Amino Acids", John Wiley & Sons, Inc., New York, 1961.

A substantially water-immiscible, aprotic solvent for use in this invention is defined as an aprotic solvent in which the solubility in water is not more than 5%v. For example, the solvent is a hydrocarbon, or ether solvent including acyclic, alicyclic or aromatic materials. Preferably, at the reaction temperature the solvent has a boiling point below about 150°C (and does not interfere with the reaction). For example, suitable solvents are alkanes containing from 5 to 10 carbon atoms such as n-pentane, n-hexane, n-heptane, n-octane, n-nonane, n-decane and their isomers. Petroleum fractions rich in alkanes are also suitable, for example, gasoline with a boiling range at atmospheric pressure of between 40° and 65°C, between 60° and 80°C or between 80° and 110°C. Petroleum ether is also suitable. Cyclohexane and methylcyclohexanes are examples of useful cycloalkanes containing from 6 to 8 carbon atoms. Aromatic hydrocarbon solvents can contain from 6 to 10 carbon atoms, for example, benzene, toluene, o-, m- and p-xylene, the trimethylbenzenes, p-ethyltoluene and the like. Useful ethers include diethyl ether, diisopropyl ether, methyl-t-butyl ether and the like. Preferably, the solvent is an aromatic hydrocarbon, especially toluene, diisopropyl ether or diethyl ether or mixtures thereof (e.g. 25/75 of diethyl ether/toluene).

The reaction to prepare the optically-active S-alpha-cyano-3-phenoxybenzyl alcohols is suitably conducted by adding the aldehyde and solvent to the cyclo(D-phenylalanyl-D-histidine) catalyst, dispersing (mechanical grinding or agitating the mixture, e.g. by stirring), adding hydrogen cyanide and maintaining the reaction conditions for an amount of time to effect the formation of the optically-active alpha-hydroxynitrile. That is, preferably, the hydrogen cyanide is introduced concurrently with, subsequent to or rapidly followed by the solvent and/or aldehyde to increase the conversion and stereoselectivity. The presence of cyanide ions has an adverse effect on the catalyst in this reaction and competing racemization is reduced by protecting the catalyst from cyanide ions. The forming and maintaining of a well dispersed but not necessarily homogeneous-like reaction mixture are useful. Separation and recovery of the optically-active alcohol product are achieved by conventional techniques, including extraction and the like.

The temperature of the reaction as well as the pressure can vary. At normal pressures, the temperature is from about -10°C to about 80°C, more or less. Preferably, ambient temperatures of about 5°C to about 35°C are convenient to give good yield, rate of reaction and enantiomeric excess of the desired optically-active product, the lower temperature of 5°C giving good results.

The amount of catalyst used in making the optically-active alpha-hydroxynitriles, e.g. S-alpha-hydroxynitrile, can vary. For example, it can be used in the range of from about 0.1 to about 10 mole percent and, preferably 0.1 to about 5 mole percent based upon the weight of the aldehyde present, especially about 1.5 to about 7.5 mole percent. The catalyst is preferably well dispersed in the reaction mixture.

When the catalysts are prepared by conventional methods in the presence of solvent (e.g. water), they can, if solid, also contain solvent (e.g. water) of crystallization. The optically-active cyclo(D-phenylalanyl-D-histidine) dipeptide catalyst of the invention thus includes the presence or absence of solvent (e.g. water) of crystallization when solid.

The non-symmetrical ketenes used to prepare the cyanomethyl esters are generally known in the art. Ketenes used in the present invention can be prepared by treating the corresponding acid halide with a tertiary amine.

Suitable tertiary amines include alkyl, aryl or heterocyclic tertiary nitrogen base including mono- or polyamines and the like. Preferably, the tertiary amine is an amien in which any alkyl groups contain from 1 to 10 carbon atoms, any aryl or aralkyl groups contain from 6 to 20 carbon atoms and 1 to 2 hydrocarbyl rings, and any heterocyclic amines contain at least one ring nitrogen atom in a 5 or 6 membered heterocyclic ring optionally containing a sulfur or oxygen atom or another nitrogen atom, such as trimethylamine, triethylamine, tri-n-propylamine, pyridine and the like. Desirably, a tertiary amine contains three alkyl groups of 1 to 4 carbon atoms, for example: trimethylamine, tri-n-propylamine, and especially triethylamine or trimethylamine.

The reaction to prepare the non-symmetrical ketene is conducted in the presence or absence of a solvent. When a solvent is used the solvent is preferably a non-hydroxylic solvent such as hydrocarbons, chlorinated hydrocarbons, ethers and the like. For example, suitable solvents are alkanes containing from 5 to 10 carbon atoms such as n-pentane, n-hexane, n-heptane, n-octane, n-nonane, n-decane and their isomers. Petroleum fractions rich in alkanes are also suitable, for example, gasoline with a boiling range at atmospheric pressure of between 40° and 65°C, between 60° and 80°C or between 80° and 110°C. Petroleum ether is also suitable. Cyclohexane and methylcyclohexanes are examples of useful cycloalkanes containing from 6 to 8 carbon atoms. Aromatic hydrocarbon solvents can contain from 6 to 10 carbon atoms, for example, benzene, toluene, o-, m- and p-xylene, the trimethylbenzenes, p-ethyltoluene and the like. Suitable chlorinated hydrocarbons contain from 1 to 4 chlorine atoms in combination with an alkane chain containing from 1 to 4 carbon atoms or with a benzene ring, for example, carbon tetrachloride, chloroform, dichloromethane, 1,2-dichloroethane, trichloroethane, perchloroethane, chlorobenzene and 1,2- or 1,3-dichlorobenzene and the like. Ethers are generally those containing from 4 to 6 carbon atoms such as diethyl ether, methyl tert-butyl ether and diisopropyl ether and the like. Tetrahydrofuran and dioxane are also useful. Preferably, the solvent, if used, is an aromatic hydrocarbon, especially toluene.

In the preparation of the non-symmetrical ketene, the molar ratio of the starting materials can be varied widely. For example, the molar ratio of acid halide to base is from about 10:1 to about 1:10, and preferably from about 5:1 to about 1:5. However, it is desirable to have a molar excess of base to acid halide. Therefore, a molar ratio of acid halide to base is conveniently from about 1:1.2 to about 1:2 and desirably from about 1:1 to about 1:5.

In the preparation of the non-symmetrical ketene, the temperature can be varied widely. At normal pressure, for example, the temperature of reaction can be varied and is suitably, for example, from about 10°C to 40°C more or less, although higher temperatures of about 75° to about 95°C have been found very useful.

Separation and recovery of the product non-symmetrical ketene are achieved by conventional methods, including crystallization and the like.

This process of the invention is useful for preparing non-symmetrical ketenes from any acid halides which do not contain substituted groups which would react with the base. For example, the acid halide can be that of an acyclic, alicyclic, aromatic or heteroaromatic acid. Preferably, the acid halide has the formula V

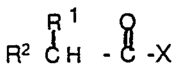 V

wherein X is the halogen atom, such as chlorine or bromine, $R^1$ and $R^2$ each independently is an alkyl, aralkyl, alkoxy, aryloxy, alkylthio, alkylsulfonyl, arylthio, or arylsulfonyl group containing from 1 to 10 carbon atoms or a cycloalkyl group containing 3 to 7 ring carbon atoms, or when taken together with the carbon atom to which they are attached form a non-symmetrical cycloalkyl group containing 3 to 7 ring carbon atoms; $R^2$ is also an alkenyl or alkynyl containing from 2 to 10 carbon atoms; a naphthyl group; a phenyl group; a heterocyclic group containing 5 or 6 ring atoms, one of which is oxygen, sulfur or nitrogen, and the remainder are carbon atoms, or is an amino group disubstituted by acyl, alkyl containing up to 10 carbon atoms, or a phenyl group. The $R^1$ and $R^2$ groups can be optionally substituted by one or more of halogen of atomic numbers 9 to 35, an alkyl, haloalkyl or cycloalkyl group containing up to 7 carbon atoms, alkenyl or haloalkenyl group of 2 to 4, haloalkoxy or alkoxy group of 1 to 4 carbon atoms, haloalkylthio or alkylthio group of 1 to 4 carbon atoms or equivalent kinds of substituents.

One class of acid halides are halides of pyrethroid acids, including those of U.S. patents 4,062,968 and 4,199,595. Examples of such acid halides include those having the formula V in which $R^1$ is isopropyl or cyclopropyl; $R^2$ is an alkyl group containing 1 to 6 carbon atoms; an alkenyl group containing 2 to 6 carbon atoms; a naphthyl group, a phenyl group or a (benzyloxycarbonyl)phenylamino group, each optionally ring-substituted by one or more of halogen, alkyl, haloalkyl, alkoxy, haloalkoxy in which the halogens are bromine, chlorine or fluorine and the alkyl groups contain 1 or 4 carbon atoms. For example, the acid halide is isopropyl(4-chlorophenyl)acetyl chloride, isopropyl(4-(difluoromethoxy)phenyl)acetyl chloride, isopropyl((4-(trifluoromethyl)-3-chlorophenyl)(benzyloxycarbonyl)amino)acetyl chloride, and the like.

Preferably, in formula V, $R^1$ is isopropyl and $R^2$ is a phenyl group optionally substituted by halogen, an alkyl or haloalkyl group of 1 to 4 carbon atoms or an alkoxy or haloalkoxy group containing 1 to 4 carbon atoms, preferably at the para position, especially useful are 4-chlorophenyl, 4-(difluoromethoxyphenyl), 4-methylphenyl, 4-tert-butylphenyl and the like.

Many of the non-symmetrical ketenes of the invention are known in the art per se, for example, (4-chlorophenyl)isopropylketene, as in U.S. patent 4,199,527. Some other non-symmetrical ketenes are believed to be generally known, but specific species may be novel, for example, including (4-(difluoromethoxy)phenyl)isopropylketene.

Another embodiment of the invention is directed to a process for the preparation of an optically-active cyanomethyl ester or a mixture enriched therein which comprises treating an alpha-chiral (optically-active) carboxylic acid halide or reactive derivative thereof, or mixture enriched therein, with an optically-active, optionally substituted S-alpha-cyano-3-phenoxybenzyl alcohol or mixture enriched therein which has been prepared as described above.

This process of the invention is useful for preparing esters from any optically-active acid halides (which do not contain substituted groups which would react with the base, when present). For example, the acid halide can be that of an acyclic, alicyclic, aromatic or heteroaromatic acid.

The reaction is conducted in the absence of a solvent or in the presence of an organic solvent, which is suitably selected from the same kinds of non-hydroxylic solvents described above for the process for the preparation of esters from treating a non-symmetrical ketene. Preferably, the solvent is an aromatic solvent, such as toluene.

The reaction with acid halide is preferably conducted in the presence of a hydrogen halide acceptor, which is suitably a tertiary amine such as triethylamine or pyridine, added slowly with agitation and usually after the other reactants are well mixed.

The reaction to prepare optically-active cyanomethyl esters from the S-alcohols and alpha-chiral carboxylic acid halides or reactive derivative thereof is also suitably conducted under phase-transfer conditions in the presence of a phase-transfer catalyst, which effectively facilitates the transfer of ions or other reactive or functional chemical species across the phase interface as in heterogeneous systems. Non-limiting examples of phase-transfer catalysts include certain organic quaternary salts of Group VA elements of the Periodic Table of Elements, e.g. of nitrogen, phosphorus, arsenic, antimony and bismuth.

The preferred phase-transfer catalysts are tetra-n-butyl phosphonium chloride, tri-n-butyl n-octyl phosphonium bromide, hexadecyl tributyl phosphonium bromide, benzyl triethyl ammonium chloride, benzyl triethyl ammonium bromide, trioctyl ethyl ammonium bromide, tetraheptyl ammonium iodide, triphenyl decyl phosphonium iodide, tribenzyl decyl ammonium chloride, tetranonyl ammonium hydroxide, tricaprylyl methyl ammonium chloride and dimethyl dicoco ammonium chloride. The last two catalysts are manufactured by General Mills Company, Chemical Division, Kankakee, Ill., and are alternatively designated by the names "Aliquat 336®" and "Aliquat 221®", respectively.

In the preparation of the esters, the molar ratio of the starting materials can be varied widely. For example, the molar ratio of acid halide to alcohol is from about 10:1 to about 1:10, and preferably from about 5:1 to about 1:5. However, it is desirable to have a molar excess of acid halide to alcohol. Therefore, a molar ratio of alcohol to acid halide is desirably from about 1:1 to about 1:5 and conveniently from about 1:1 to about 1:1.2.

In the preparation of the ester, the temperature can be varied widely. At normal pressure, for example, the temperature of reaction can be varied for example, from about 0°C to about 70°C, but is preferably from about 10°C to 40°C more or less.

Separation and recovery of the product ester are achieved by conventional methods, including crystallization and the like.

This embodiment of the process of the invention is useful for preparing esters from any acids halide, or reactive derivative thereof, which do not contain substituted groups which would react with the base. For example, the acid halide or reactive derivatives thereof are conventionally known in the art and include an acyclic, alicyclic, aromatic or heteroaromatic acids and reactive derivatives thereof and preferably has the formula VI

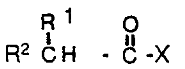 VI

wherein X is a reactive derivative of the corresponding carboxylic acid; $R^1$ and $R^2$ each independently is an alkyl, aralkyl, alkoxy, aryloxy, alkylthio, alkylsulfonyl, arylthio, or arylsulfonyl group containing from 1 to 10 carbon atoms or a cycloalkyl group containing 3 to 7 ring carbon atoms, or when taken together with the carbon atom to which they are attched form a non-symmetrical cycloalkyl group containing 3 to 7 ring carbon atoms; $R^2$ is also an alkenyl or alkynyl containing from 2 to 10 carbon atoms; a naphthyl group; a phenyl group; a heterocyclic group containing 5 or 6 ring atoms, one of which is oxygen, sulfur or nitrogen, and the remainder are carbon atoms, or is an amino group disubstituted by acyl, alkyl containing up to 10 carbon atoms, or a phenyl group. The $R^1$ and $R^2$ groups can be optionally substituted by one or more of halogen of atomic numbers 9 to 35, an alkyl, haloalkyl or cycloalkyl group containing up to 7 carbon atoms, alkenyl or haloalkenyl group of 2 to 4, haloalkoxy or alkoxy group of 1 to 4 carbon atoms, haloalkylthio or alkylthio group of 1 to 4 carbon atoms or equivalent kinds of substituents. As reactive derivatives halides are preferred, e.g. of formula VI above in which X is chlorine or bromine.

One class of acid halides are of pyrethroid acids, including those of U.S. patents 4,024,163, 4,062,968, 4,220,591, 3,835,176, 4,243,819, 4,316,913 and 4,199,595. Examples of such acid halides or reactive derivatives thereof include those having the formula VI in which $R^1$ is isopropyl or cyclopropyl; $R^2$ is an alkyl group containing 1 to 6 carbon atoms; an alkenyl group containing 2 to 6 carbon atoms; a naphthyl group, a phenyl group or a (benzyloxycarbonyl)phenylamino group, each optionally ring-substituted by one or more of halogen, alkyl, haloalkyl, alkoxy, haloalkoxy in which the halogens are bromine, chlorine or fluorine and the alkyl groups contain 1 or 4 carbon atoms, or $R^1$ and $R^2$ together with the carbon atom to which they are attached form a cyclopropyl group of the formula

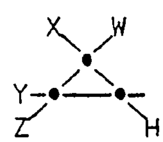

in which W, X, Y and Z each independently is a hydrogen atom, a halogen atom of atomic numbers 9 to 35 or an alkyl group containing 1 to 4 carbon atoms or Y and Z each independently is an alkyl group containing 1 to 4 carbon atoms, W is a hydrogen atom and X is pentahaloethyl, dihalovinyl, isobutenyl, perhalomethylvinyl, 2-phenyl-2-halovinyl 2-phenyl-1,2,2-trihaloethyl group or alkoxyiminomethyl or (-(cycloalkyl)alkoxy)iminomethyl of 1 to 10 carbon atoms. For example, the acid halide is isopropyl(4-chlorophenyl)acetyl chloride, isopropyl(4-(difluoromethoxy)phenyl)acetyl chloride, isopropyl((4-trifluoromethyl-3-chlorophenyl)(benzyloxycarbonyl)amino)acetyl chloride, 2,2-dimethyl-3-(2,2-dichlorovinyl)-cyclopropanecarbonyl chloride, 2,2-dimethyl-3-(2,2-dibromovinyl)cyclopropanecarbonyl chloride, 2,2-dimethyl-3-(1,2-dibromo-2,2-dichloroethyl)cyclopropanecarbonyl chloride, 1-(4-ethoxyphenyl)-2,2-dichlorocyclopropanecarbonyl chloride, 2,2-dimethyl-3-(2-(trifluoromethyl)-2-chlorovinyl)cyclopropanecarbonyl chloride, 2,2-dimethyl-3-((isobutoxyimino)methyl)cyclopropanecarbonyl chloride, 2,2-dimethyl-3-(-(neopentoxyimino)methyl)cyclopropanecarbonyl chloride, 2,2-dimethyl-3-(((cyclobutyl)methoxyimino)-methyl)cyclopropanecarbonyl chloride, or chrysanthemyl chloride, and the like.

Preferably, in formula VI, $R^1$ is isopropyl and $R^2$ is a phenyl group optionally substituted by halogen, an alkyl or haloalkyl group of 1 to 4 carbon atoms or an alkoxy or haloalkoxy group containing 1 to 4 carbon atoms, preferably at the para position, especially useful are 4-chlorophenyl, 4-(difluoromethoxyphenyl), 4-methylphenyl, 4-tert-butylphenyl and the like.

In one embodiment of the invention, an S-alpha-cyano-3-phenoxybenzyl alcohol or mixture enriched therein is treated with an S-alphaisopropylphenylacetic acid chloride or an optionally-substituted chiral cyclopropanecarboxylic acid chloride to give an optically-active cyanomethyl ester or a mixture enriched therein.

The cyanomethyl esters for which the optically-active form is prepared by one or more embodiments of the process of the invention, i.e. of formula I

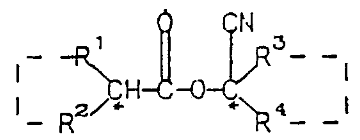

I

are generally known in the art, including from Francis et al., J. Chem. Soc., 95, pages 1403-1409 (1909) and the like, and in the optical forms, including U.S. patents 4,151,195, 4,239,737, 4,328,167 and 4,133,826, and British patent 2,014,137 and the like. Any of the alpha-cyanomethyl esters prepared can be hydrolyzed to their corresponding acids by conventional hydrolysis methods known in the art. Preferably, the product optically-active ester is S-alpha-cyano-3-phenoxybenzyl S-alpha-isopropyl(p-chlorophenyl)acetate, S-alpha-cyano-3-phenoxybenzyl S-alpha-isopropyl(p-difluoromethoxy)phenyl)acetate, S-alpha-cyano-3-phenoxybenzyl (1R,cis)-3-(2,2-dichlorovinyl)-2,2-dimethylcyclopropanecarboxylate, S-alpha-cyano-3-phenoxybenzyl (1R,cis)-3-(2,2-dibromovinyl)-2,2-dimethylcyclopropanecarboxylate, S-alpha-cyano-3-phenoxybenzyl (1R,cis)-3-(1,2-dibromo-2,2-dichloroethyl)-2,2-dimethylcyclopropanecarboxylate, S-alpha-cyano-3-phenoxybenzyl (1R,cis)-2,2-dimethyl-3-(neopentoxyiminomethyl)cyclopropanecarboxylate, and the like.

## Illustrative Embodiments

### Embodiment 1 - N-(Benzyloxycarbonyl)-D-phenylalanine

A 15.0 g sample of D-phenylalanine was dissolved in 45 ml of aqueous solution containing 7.26 g of 50% sodium hydroxide. This solution was stirred at 0-10 °C as 16.3 g of benzyl chloroformate was added rapidly in portions. The resulting reaction was mildly exothermic, and shortly after addition, solids precipitated. An additional 45 ml of water and 3.63 g of 50% sodium hydroxide were added, causing most of the solids to redissolve. The reaction mixture was stirred for 20 minutes and then acidified with 6 N hydrochloric acid. The resulting solids were filtered, washed with water and then with hexane, and dried by suction and then under vacuum to give 47 g of white solids. These solids dissolved in ether were washed twice with 1 N hydrochloric acid and then with water, dried over $MgSO_4$ and stripped to 35 °C at 2.5 mm Hg to give 27.7 g of the desired product as a colorless oil.

Embodiment 2 - N-(Benzyloxycarbonyl)-D-phenylalanine, p-nitrophenyl Ester

A 300 ml three-neck flask with stirrer and dropping funnel was charged under a nitrogen atmosphere with 27 g of the acid of Embodiment 1 above in 135 ml of pyridine, followed by 13.2 g of p-nitrophenol. The resulting solution was cooled to 0° to 10° C as 14.6 g of phosphorus oxychloride was added. The resulting mixture was warmed to 25° C, stirred for 15 minutes, then poured into 300 ml of ice water. Filtration of the resulting solid, followed by washing with water and drying by suction, gave 33 g of product. This was crystallized from 340 ml of hot ethyl alcohol with chilling to -5° C. The product was filtered, washed with chilled ethyl alcohol, then with hexane, and sucked dry to give 28.7 g of the desired product, m.p. 122.5-124.5° C, $[\alpha]_D^{23}$ +24.7 (c 2.0, dimethylformamide).

Embodiment 3 - N-(Benzyloxycarbonyl)-D-phenylalanyl-D-histidine Methyl Ester

To a stirred solution of 5.0 g of D-histidine methyl ester hydrochloride in 40 ml of methylene chloride was added 4.18 g of triethylamine followed by 8.27 g of the nitrophenyl ester prepared as in Embodiment 2 above. The reaction mixture immediately became bright yellow and solids began to precipitate. The reaction mixture was stirred for 2 hours, then stored overnight at -10° C. The reaction mixture was rewarmed to room temperature, and 0.6 ml of triethylamine was added. Then, 490 mg of the D-histidine methyl ester hydrochloride was added, and stirring was continued for 2 hours. The reaction mixture was washed with 20 ml of water, then twice with 20 ml of 10% ammonium hydroxide, and then twice with 20 ml of water. All the washes were back-extracted serially with 20 ml of methylene chloride, and the combined organic phases was dried with $MgSO_4$ and stripped to 100 ml, filtered through silica, followed by 25 ml of 20% methanol in ethyl acetate. The resulting eluate was stripped to 40 ml and diluted to 120 ml with diethyl ether; the pricipitated solid was filtered, washed with diethyl ether, and dried by suction to give 5.66 g of the desired product as a white solid, m.p. 114.5-117° C $[\alpha]_D^{20}$ -55.5 (c 2 in $CHCl_3$).

Embodiment 4 - Cyclo(D-phenylalanyl-D-histidine)

5.60 g of methyl ester of Embodiment 3 above was stirred and hydrogenated in 100 ml of methanol over 220 mg of 10% palladium on carbon at atmospheric pressure. After 3 hours, solids began to precipitate; an additional 2.5 ml of methanol was added to facilitate stirring. After 7 hours, an additional 280 ml of methanol was added as the mixture was heated to reflux. The mixture was filtered hot, and the filtrate was stripped to a gel-mush, which was mixed with 100 ml of diethyl ether. The resulting solid was filtered, washed with diethyl ether, and dried by suction and then under high vacuum at 35° C to give 3.29 g of the desired product as an off-white powder, $[\alpha]_D^{23}$ = +68.5 (c 2.0 in $CH_3COOH$).

Embodiment 5 - (4-Chlorophenyl)isopropylketene

To a solution of 2.31 g of isopropyl(4-chlorophenyl)acetyl chloride in 10 ml of methylene chloride was added in one portion 1.5 g of triethylamine. After 18 hours, 15 ml of heptane was added to the mixture and the triethylamine hydrochloride was removed by filtration. The filtrate was stripped and 10 ml of heptane was added and the resulting mixture was filtered and stripped to give a yellow residue, which was dissolved in 5 ml heptane for GLC analysis. The resulting solution was distilled through a Bantam-ware short-neck head from an oil bath at 125-150° C and head temperature of 110-100° C at 0.2-0.05 mm to give 0.95 g of distillate and 0.81 g of gum. The distillate was crystallized twice from 2 volumes of hexane at -80° C. The solid was melted and stripped to about 40° C at 0.5 mm to give 0.42 g of the desired product as a yellow liquid.

Embodiment 6 - (4-Chlorophenyl)isopropylketene

A sample of 53.2 g of isopropyl(4-chlorophenyl)acetic acid was treated with 21.5 ml of thionyl chloride in a 500 ml flask and heated slowly to 80° C and maintained at 80° C for 20 minutes. The reaction mixture was allowed to stand at room temperature for 2 days. The volatiles were stripped to 75° C at 0.5 mm Hg. The resulting yellow liquid was diluted with 250 ml of methylene chloride followed by addition of 38.0 g of

triethylamine. The mixture was stirred until triethylamaine hydrochloride began to precipitate after 30 minutes. After 16 hours, the reaction mixture was filtered and solid triethylamine hydrochloride was washed with heptane. Most of the solvent was stripped from the filtrate by rotary evaporation at 50° C. The residue was diluted with 75 ml of heptane and additional triethylamine hydrochloride was removed by filtration as above. The filtrate was restripped and rediluted with 75 ml heptane and refiltered with the aid of 25 ml of heptane. The filtrate was cooled in dry ice, seeded and crystallized. The resulting crystals were filtered with a filter stick and washed with chilled heptane. The filtered solids were melted, diluted with one-half volume heptane, crystallized at -80° C and the collected solid was melted and stored at -80° C. The filtrate solution was warmed, stripped of most solvent, then distilled through a Bantam ware short path head at 0.05 to 0.06 mm Hg from an oil bath at 90°-120° C. Total distillate was 14.5 g collected as a bright yellow-orange liquid at a head temperature of 60°-85° C. The distillate was crystallized from an equal volume of pentane at -80° C, filtered and washed twice with heptane as above to give, on warming, a second melt. The stripped filtrates totalling 5.79 g were crystallized as above in a 6-inch test tube and the melt was recrystallized immediately as described above to give a third melt. The three melts were combined and stripped to 50° C at 5 mm Hg to give 29.4 g of the desired ketene as a yellow liquid.

Embodiment 7 - (4-Chlorophenyl)isopropylketene

To 57.75 g of isopropyl(4-chlorophenyl)acetyl chloride was added 69.4 ml of triethylamine. The mixture was allowed to stand overnight at 20° C. The resulting mushy solid was crushed, diluted with 300 ml of redistilled hexane and filtered. The solids were washed three times with 75 ml of hexane, filtered and dried by suction with calcium chloride dried air to give 32 g triethylamine hydrochloride. The combined hexane solutions of ketene slowly deposited additional solids; the mixture was let stand at room temperature overnight with the flask wrapped in aluminum foil and filtered again to give 0.75 g of additional solids. The solvent was removed from the filtrate by rotary evaporation, then taken briefly to 1 mm Hg. To the mixture was added 500 ml of hexane, and after filtration, the filtrate was stripped to a yellow oil. This oil was distilled through a Bantam-ware short path head at 0.5 mm Hg to give 28.61 g of the desired ketene as a yellow liquid, $d^{20}$ 1.10.

Embodiment 8 - S-alpha-Cyano-3-phenoxybenzyl Alcohol

A 100 ml three-neck Bantam-ware flask was charged with 43 mg of cyclo(D-phenylalanyl-D-histidine) and put under a nitrogen atmosphere. Then, 3.51 ml of hydrogen cyanide was added by syringe causing the catalyst to swell and become a gel. After 5 minutes, 30 ml of toluene was added, causing additional catalyst to precipitate. 5.95 g of 3-phenoxybenzaldehyde was added all at once. The reaction mixture was stirred for 4.75 hours and then quenched with 20 ml of water containing 10 drops of concentrated hydrochloric acid. The toluene solution was separated, washed twice with water, and diluted to 50 ml with toluene for analysis, which showed 80% S-alpha-cyano-3-phenoxybenzyl alcohol isomer was produced.

Embodiment 9 - S-alpha-Cyano-3-phenoxybenzyl Alcohol

The reaction of Embodiment 8 above was repeated using 171 mg of cyclo(D-phenylalanyl-D-histidine). At intervals, 0.25 ml samples were removed and examined by gas liquid chromatography as follows:

| Time | % Conversion of Aldehyde |
|---|---|
| 35 minutes | 20 |
| 2 hours | 76 |
| 6.5 hours | 95 |

After 7 hours, the reaction mixture was quenched by addition of 10 ml of 1 N hydrochloric acid. The organic phase was separated and washed twice with water, dried over $MgSO_4$, filtered and stored at -10° C. The filtrate was diluted to 50 ml with toluene and the optical rotation was determined to be -1.54° at 21° in 1 dm cell. A sample of the product was acetylated with p-nitrophenylacetic anhydride and the sterioisomer

ratio was determined by HPLC on a chiral Pirkle column to be 71% S-alpha-cyano-3-phenoxybenzyl alcohol and 29% R-alpha-cyano-3-phenoxybenzyl alcohol.

Embodiment 10 - S-alpha-Cyano-3-phenoxybenzyl Alcohol

Two small round-bottom flasks having magnetic stirrers and septum covers were each charged with 22.5 mg of cyclo(D-phenylalanyl-D-histidine) and put under nitrogen. A sample of 0.98 ml of hydrogen cyanide was diluted to 25 ml with toluene, and 5 ml of the solution was added via syringe to each flask. After about 5 minutes, 0.87 ml of 3-phenoxybenzaldehyde (POAL) was added to each flask. Flask No. 1 was stirred in an oil bath at 35°C and flask No. 2 was stirred in a water bath at 24-26°C. The results of these experiments are below.

| | Flask No. 1 | | | Flask No. 2 | | |
|---|---|---|---|---|---|---|
| Time, hr | POAL, % | α-Hydroxy-nitrile, % | R/S | POAL, % | α-Hydroxy-nitrile, % | R/S |
| 0.5 | 19 | 81 | 8.7/91.3 | 18 | 82 | 15.8/84.2 |
| 1 | 12 | 88 | - | 13 | 87 | 14.4/85.6 |
| 2 | 12 | 88 | - | 12 | 88 | - |
| 4 | 10 | 90 | 11.4/88.6 | 12 | 88 | 19 0/81.0 |
| 8 | 10 | 90 | - | 13 | 87 | - |

Embodiment 11 S-alpha-Cyano-3-phenoxybenzyl Alcohol

A reaction was conducted by contacting 0.0099 m/kg cyclo(D-phenylalanyl-D-histidine) with 0.99 m/kg of 3 phenoxybenzaldehyde followed by 2 2 m/kg of hydrogen cyanide and 190 ppm water. The reaction was conducted in toluene at 25°C. The product obtained with 93% conversion of aldehyde was 88% S-alpha-cyano-3-phenoxybenzyl alcohol isomer.

Embodiment 12 S-alpha-Cyano-3-phenoxybenzyl Alcohol

To 0.2933 g of cyclo(D-phenylalanyl-D-histidine) in 15 ml of diethyl ether at 25°C was added 2.907 g of 3-phenoxybenzaldehyde followed by 0.940 g of hydrogen cyanide. After 2 hours, 94% of the 3-phenoxybenzaldehyde had been converted and the product had an S-alpha-cyano-3-phenoxybenzyl alcohol enantiomeric excess of 84%. After 4 to 20 hours, 99.4% of the 3 phenoxybenzaldehyde had been converted and the product had an S-alpha-cyano-3-phenoxybenzyl alcohol enantiomeric excess of 67-68%.

Embodiment 13 S-alpha-Cyano-3-phenoxybenzyl Alcohol

A reaction was conducted by treating 0.0200 g of cyclo(D-phenylalanyl-D-histidine) at 25°C under nitrogen with 1.4037 g of 3-phenoxybenzaldehyde to disperse the catalyst followed by injecting 2.1812 g of toluene with 13.65 w% hydrogen cyanide. After 2.7 hours, 93% of the 3-phenoxybenzaldehyde was converted and the product had an S-alpha-cyano-3-phenoxybenzyl alcohol enantiomeric excess of 77%.

Embodiment 14 - S-alpha-Cyano-3-phenoxybenzyl Alcohol

A reaction was conducted at 25°C by contacting 0.0519 g of cyclo(D-phenylalanyl-D-histidine) in 9.32 ml of diethyl ether with 1.811 g of 3-phenoxybenzaldehyde followed by 0.617 g of hydrogen cyanide. After 3.6 hours, 99.4% of the 3-phenoxybenzaldehyde had been converted and the product had an S-alpha-cyano-3-phenoxybenzyl alcohol enantiomeric excess of 72%.

Embodiment 15 - S-alpha-Cyano-3-phenoxybenzyl S-Isopropyl(4-chlorophenyl)acetate

Two 1 dram vials with toluene were each charged with 1 ml of solution containing 0.135 g of S-alpha-cyano-3-phenoxybenzyl alcohol followed by 0.504 ml of S-isopropyl(4-chlorophenyl)acetyl chloride solution in toluene.

To the first vial was added with stirring 0.07 ml of 2,6-lutidine. The resulting mixture became warm immediately and precipitated solids. Stirring was continued for 10 minutes with no further change. The mixture was washed successively with water, dilute hydrochloric acid and water, and dried ($MgSO_4$). The resulting oil contained 75.5% of the S-alpha-cyano-3-phenoxybenzyl S-isopropyl(4-chlorophenyl)acetate isomer.

To the second vial was added with stirring 0.083 ml of triethylamine. An immediate reaction occurred, and the product was recovered as described above to give an oil containing 72.0% of S-alpha-cyano-3-phenoxybenzyl S-isopropyl(4-chlorophenyl)acetate isomer.

Embodiment 16 - S-alpha-Cyano-3-phenoxybenzyl (1R,cis)-2,2-Dimethyl-3-(2,2-dichlorovinyl)-cyclopropanecarboxylate

To a solution of 1 g of (1R,cis)-2,2-dimethyl-3-(2,2-dichlorovinyl)cyclopropanecarboxylic acid dissolved in 25 ml of methylene chloride was added 0.5 g of thionyl chloride followed by a few drops of dimethylformamide. The reaction mixture was refluxed overnight, and the solvent was removed under pressure at 40° C. The residue was dissolved in 80 ml benzene and 0.4 g of S-alpha-cyano-3-phenoxybenzyl alcohol (90% enantiomeric excess to S-isomer) in 2 ml of benzene was added followed by 0.5 ml of pyridine in 2 ml of benzene. The reaction mixture was stirred for 1.5 hours. The resulting solution was poured into water, extracted with ether, the ether layer was washed with dilute hydrochloric acid, then with sodium bicarbonate solution. The organic layer was washed with water, dried ($MgSO_4$) and condensed to give a yellow oil, which was chromatographed to give 0.53 g of the desired product, $[\alpha]_D^{20} = 26.55^\circ$ ($CH_2Cl_2$ 0.558g/ml).

Embodiment 17 - S-alpha Cyano-3-phenoxybenzyl S-Isopropyl(4-chlorophenyl)acetate

A 1 dram vial was charaged with 1 ml of a solution of alpha-cyano-3-phenoxybenzyl alcohol having an R/S ratio of ca 72:28, and 0.121 ml of (4-chlorophenyl)isopropylketene. Then, 7.5 mg of cyclo(D-phenylalanyl-D-histidine) was added. The reaction mixture was stirred at ambient temperature for 24 hours to give a colorless product containing a white, insoluble floc. The reaction mixture was centrifuged to remove solids, the liquid phase was decanted and washed with 1 N hydrochloric acid and twice with water, dried with $MgSO_4$, filtered and diluted to 1 ml with solvent for analysis. The desired product had by Pirkle column analysis a ratio of 55.1% S-alpha-cyano-3-phenoxybenzyl S-isopropyl(4-chlorophenyl)acetate and 16.6% R-alpha-cyano-3-phenoxybenzyl R-isopropyl(4-chlorophenyl)acetate.

Embodiment 18 - S-alpha-Cyano-3-phenoxybenzyl S-Isopropyl(4-chlorophenyl)acetate

A 1 dram vial was charged with 1 ml of alpha-cyano-3-phenoxybenzyl alcohol solution as described in Embodiment 17 followed by 0.121 ml of (4-chlorophenyl)isopropylketene and then 7.5 mg of cyclo(D-phenylalanyl-D-histidine). The resulting mixture was stirred at ambient temperature for 20 hours to give a colorless product liquid containing a white floc. This reaction product mixture was centrifuged and the insoluble cake of gel was washed and centrifuged 4 times with 1 ml portions of hexane. The combined organic extracts was washed with dilute hydrochloric acid and water, dried, stripped to below 1 ml and then diluted to 2 ml in toluene. The desired product had by Pirkle column analysis a ratio of 63.0% S-alpha-cyano-3-phenoxybenzyl S-isopropyl(4-chlorophenyl)acetate and 12.5% R-alpha-cyano-3-phenoxybenzyl R-isopropyl(4-chlorophenyl)acetate.

Embodiment 19 - S-alpha-Cyano-3-phenoxybenzyl S-Isopropyl(4-chloropheny)acetate

The catalyst gel recovered from Embodiment 18 above was washed with 1.5 ml of hexane and charged to a 1 dram vial. The vial was charged with 1 ml of S-alpha-cyano-3-phenoxybenzyl alcohol solution as described in Embodiment 14 and 0.121 ml of (4-chlorophenyl)isopropylketene. The reaction mixture was stirred for 16 hours and then extracted 5 times with 1 ml hexane, and the combined extracts was stripped and diluted to 2 ml in toluene for analysis. The desired product had by Pirkle column analysis a ratio of 62.4% S-alpha-cyano-3-phenoxybenzyl S-isopropyl(4-chlorophenyl)acetate and 14.1% R-alpha-cyano-3-phenoxybenzyl R-isopropyl(4-chlorophenyl)acetate.

Embodiment 20 - S-alpha-Cyano-3-phenoxybenzyl S-isopropyl(4-chlorophenyl)acetate

A 0.5 dram vial containing a magnetic stirring bar and 4 mg of cyclo(D-phenylalanyl-D-histidine) was filled with nitrogen and capped with a septum cap. Into this vial was injected 0.174 ml of 3-phenoxybenzaldehyde followed by 0.044 ml of hydrogen cyanide. After 5 minutes stirring, 0.18 ml of (4-chlorophenyl)-isopropylketene was added. After 2 days, the reaction mixture was diluted with toluene, washed with 1 N hydrochloric acid, water, dried ($MgSO_4$) and filtered. A solution thereof in 1 ml toluene was analyzed and determined to be enriched in the desired material.

Embodiments 21-103

A vial was charged with 13.5% w/v of racemic alpha-cyano-3-phenoxybenzyl alcohol solution in 0.6 M of toluene followed by a 5% w/v excess of (4-chlorophenyl)isopropylketene and 4 m% of a catalyst. The resulting mixture was stirred at 25 °C for a period of time until the reaction had essentially gone to completion or was terminated.

The catalyst used and the predominant isomer are set forth in the Table below in which the symbols used to describe the catalyst are standard in peptide chemistry, e.g. as set forth in Schroder and Lubke, "The Peptide", Vol. II, pages XI-XXVI (1966); and the isomers are Aα = S-acid S-alcohol isomer, Aβ = S-acid R-alcohol isomer, Bα = R-acid S-alcohol isomer and Bβ = R-acid R-alcohol isomer.

Table

| Embodiment | Catalyst | Time (hours) | % Predominant Isomer |
|---|---|---|---|
| 21 | cyc(2-pyridylala-L-Phe) | 4 | Bα =29.8 |
| 22 | cyc(D-Phe-L-His) | <28 | Bβ =38.0 |
| 23 | cyc(L-Trp-L-Trp) | >36 | Bβ =28.1 |
| 24 | Z-L-Leu-L-His-Me ester | 6.5 | Aα =32.4 |
| 25 | D-Histidine (free base) | >36 | Bβ =30.0 |
| 26 | D-His-Me ester · 2 HCl | 30 | Aα =29.3 |
| 27 | L-Phe | >30 | Aβ =30.0 |
| 28 | Ser-Me ester.HCl | >36 | Aα,Bβ=27.0 each |
| 29 | cyc(L-Leu-L-His) | 30 | Aα =29.4 |
| 30 | cyc(Gly-L-Trp) | >36 | Bβ =36.7 |
| 31 | cyc(D-Phe-D-Trp) | >52 | Aβ =36.7 |
| 32 | cyc(D-Phe-L-His) | <36 | Bβ =36.4 |
| 33 | cyc(L-Phe-L-Phe) | >52 | Aβ,Bα=27.8 each |
| 34 | cyc(L-Phe-L-Met) | >52 | Bβ =31.8 |
| 35 | cyc(D-Phe-D-His) | >8 <22 | Aα =32.6 |
| 36 | N-Acetyl-L-His·$H_2O$ | 30 | Bβ =30.6 |
| 37 | Z-D-Phe-L-His Me ester | 28 | Bβ =28.5 |
| 38 | cyc(L-HomoPhe-L-His) | <18 | Aα =29.9 |
| 39 | cyc(L-Phe-L-3-Me-His) | 1.5 | Bβ =35.8 |
| 40 | $(L\text{-}Phe)^4 . 3H_2O$ | >100 | Bα =27.0 |
| 41 | BOC-D-Phe | 6.5 | Aβ,Bα=29.9 each |
| 42 | N-Acetyl-L-Trp | 120 | Bα =29.2 |
| 43 | N-Acetyl-L-Trp Et ester | 120 | Aβ =30.9 |
| 44 | (+)-*cis* 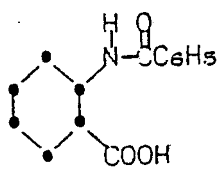 | 120 | Bα =32.6 |
| 45 | (+)-*cis* 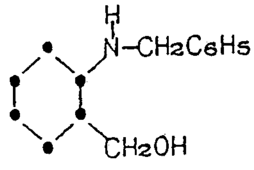 | >100 | Bα =28.7 |

Table (Continued)

| Embodiment | Catalyst | Time (hours) | % Predominant Isomer |
|---|---|---|---|
| 46 | N-alpha-Acetyl-L-Orn | >120 | Bα =26.3 |
| 47 | L-Phenylalaninol | >120 | Aα =25.5 |
| 48 | L-Carnosine | 80 | Aα =36.8 |
| 49 | L-(-)-Sparteine | 1 | Bα =28.8 |
| 50 | N-Benzyl-im-Benzyl-L-His | 0.5 | Bβ =33.0 |
| 51 | N-Z-His-p-$NO_2$-L-Phe-L-Phe-OMe | 6.5 | Aα =38.4 |
| 52 | cyc(L-Tyr-L-His) | >6 <24 | Bβ =32.7 |
| 53 | N-Z-L-His | 30 | Aα =32.4 |
| 54 | Brucine | 0.25 | Bα =32.4 |
| 55 | Nicotine | 1 | Aβ =28.8 |
| 56 | cyc(N-Ac-L-Phe-N-Ac-Gly) | 74 | Bα,Bβ=25.8 each |
| 57 | cyc(L-Val-L-His) | 80 | Aα =38.1 |
| 58 | cyc(L-Phe-Gly) | 5 days | Bα =26.8 |
| 59 | cyc(L-Phe-L-His) | <22 | Bβ =38.1 |
| 60 | L-Benzyl Hydantoin | >50 <74 | Aβ =26.9 |
| 61 | alpha-N-Me-L-His | 24 | Bβ =31.0 |
| 62 | alpha-N-Benzyl-L-His | 6.5 | Aα,Bβ=30.6 each |
| 63 | Z-L-His-L-Leu·$H_2O$ | >8 <22 | Bα =36.6 |
| 64 | N-Z-L-His-Gly | >100 | Aα =37.4 |
| 65 | Gly-L-His·HCl | 100 | Aα =27.3 |
| 66 | L-beta-Aspartyl-L-His | 100 | Bβ =31.3 |
| 67 | Z-L-His-L-Phe | >8 <22 | Aα =30.0 |
| 68 | BOC-L-Phe-L-His-OMe | >22 <72 | Aα =27.6 |
| 69 | AOC-L-Phe-L-His-OMe | >8 <22 | Aα =29.5 |
| 70 | BOC-D-PhGly-D-His-OMe | 4 | Bβ =34.2 |
| 71 | t-BOC-$N^{im}$-benzyl-L-His | 1 | Bβ =31.0 |
| 72 | t-BOC-$N^{im}$im-tosyl-L-His | >54 <72 | Bβ =27.0 |
| 73 | 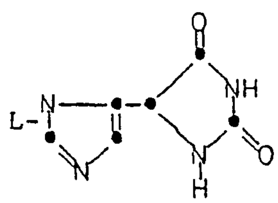 | 46 | Bβ =28.4 |
| 74 | L-Histidinol·2HCl | 28 | Aα =30.5 |
| 75 | L-beta-Imidazole Lactic acid | 22-100 | Bβ =32.2 |

Table (Continued)

| Embodiment | Catalyst | Time (hours) | % Predominant Isomer | |
|---|---|---|---|---|
| 76 | N-Z-L-Trp | >100 | Bα | =28.5 |
| 77 | N-Z-L-Trp-p-$NO_2$Ph ester | >100 | Aβ | =31.8 |
| 78 | Z-D-Phe-D-Trp-OMe | >100 | Bα | =28.5 |
| 79 | alpha-N-Benzoyl-L-Arginine | >100 | Bα | =28.8 |
| 80 | N-alpha-Benzoyl-L-Argininamide.-HCl.$H_2O$ | >100 | Bα | =28.2 |
| 81 | N-alpha-Acetyl-L-Lysine | >100 | Bα | =29.2 |
| 82 | N-alpha-Acetyl-L-Lysine-OMe.HCl | 100 | Aβ | =28.2 |
| 83 | Cyc(L-Val-Gly) | 100 | Bα | =28.6 |
| 84 | 3-Me-His-OMe.2HCl | >8 <22 | Aα | =31.2 |
| 85 | Cyc(Gly-L-His) | 30 | Bβ | =32.9 |
| 86 | Poly-L-Histidine | <46 | Aβ | =31.4 |
| 87 | Z-L-His-L-Phe-L-Phe.OEt | 6.5 | Bβ | =32.9 |
| 88 | N-alpha-Benzoyl-L-His-OMe.HCl | >10 <22 | Aα | =31.5 |
| 89 | t-BOC-L-His | >30 <46 | Aα | =30.8 |
| 90 | L-pGlu-L-His-Gly-$NH_2$ | 9 | Bβ | =35.6 |
| 91 | L-pGlu-L-His-Gly-HOAc | 0.5 | Aβ | =30.2 |
| 92 | L-beta-Ala-L-3-Me-His·$HNO_3$ | 6 | Aα | 35.3 |
| 93 | N-3,5-DNPyr-L-His | >6 <54 | Aα | =28.8 |
| 94 | OH $CH_3$<br>ℓ-Ph-CH-CHN$(Me)_2$ | 3.5 | Bβ | =26.9 |
| 95 | Z-D-Phe-NH$CH_2$- 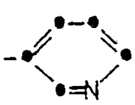 | 4.5 | Aβ | =29.2 |
| 96 | N-Benzoyl-L-His | <46 | Aα | =30.3 |
| 97 | N-ε-Acetyl-L-Lysine | >72 | Aβ | =31.4 |
| 98 | alpha-N-Benzoyl-L-Arg.OEt.HCl | >46 <54 | Aβ | =26.9 |
| 99 | Z-D-Phe-NH- 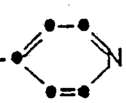 | 3.5 | Bα | =26.5 |
| 100 | L-p-Glu-L-His-L-Pro.$NH_2$ | 6 | Bβ | =35.3 |
| 101 | Cyc(L-+-Phegly-L-His) | 24 | Aα | =32.4 |
| 102 | Cyc(1-Me-L-His-L-Phe) | 10 min | Bβ | =41.3 |
| 103 | Cyc(2-naphthylala-L-His) | >6 <24 | Bβ | =34.6 |

Table (Continued)

| Embodiment | Catalyst | Time (hours) | % Predominant Isomer | |
|---|---|---|---|---|
| 104 | Z-L-Phegly-L-His-OMe | 7 | Aα | =32.8 |
| 105 | Z-L-Homophe-L-His-OMe | <23 | Bβ | =30.6 |
| 106 | cyc(D-Phe-D-His)-C(=O)-t-$C_4H_9$ | >7, <22 | Aα | =38.9 |
| 107 | Angiotensin II Pentapeptide (Tyr-Ile-His-Pro-Phe) | 48 | Aα | =34.4 |
| 108 | Z-Renin Substrate (Z-Pro-Phe-His-Leu-Leu-Val-Tyr--Ser-beta-naphthylamide) | >24 <48 | Aα | =42.1 |
| 109 | Glucagon-Hexapeptide (L-His-L-Ser-L-Glu-Gly-L-Thr-L--Phe) | >24 <48 | Aα | =31.1 |

Choice of different reaction conditions within the scope of the invention can alter the rate of reaction and amount of predominant isomer. Of course, substitution of pure optically-active R- or S-alpha-cyano-3-phenoxybenzyl alcohol or a highly enriched mixture thereof in Embodiments 21-109 above is within the scope of the invention, and increases the amount of a predominant isomer product.

Embodiment 110 - alpha-Cyano-3-phenoxybenzyl alpha-Isopropyl(4-chlorophenyl)acetate

A mixture of 2.31 g of isopropyl(4-chlorophenyl)acetyl chloride was treated with 2.78 ml of dry triethylamine similar to the procedures described in Embodiments 5-7 to give isopropyl(4-chlorophenyl)-isopropylketene in 8 ml of toluene.

A mixture of 2.00 g of 3-phenoxybenzaldehyde in 8 ml of toluene with 0.55 g of sodium cyanide was stirred under nitrogen. To this cooled solution was added 0.5 ml of water followed by slow addition of 0.86 ml of concentrated hydrochloric acid. The resulting mixture was stirred for 10 minutes at room temperature and 0.2 ml of concentrated hydrochloric acid was added dropwise. The organic phase was separated via a pipette, filtered through 3 g of magnesium sulfate using an additional 3 ml of toluene, and the total filtrate was added to the above prepared ketene solution (containing triethylamine) prechilled to -50°C. The reaction mixture was warmed to room temperature, allowed to stand 10 minutes, then washed successively with water, 5% sodium carbonate solution, water, and dilute hydrochloric acid, poured onto a column of silica gel, and eluted with 36% diethyl ether in toluene. The eluate was dried ($MgSO_4$) and stripped of solvent to give 3.98 g of a brown oil, which had an isomer ratio of 65% of the enantiomer pair S-alpha-cyano-3-phenoxybenzyl R-isopropyl(4-chlorophenyl)acetate and R-alpha-cyano-3-phenoxybenzyl S-isopropyl(4-chlorophenyl)acetate and 35% of the R,R and S,S enantiomer pair.

Embodiment 111 - alpha-Cyano-3-phenoxybenzyl Isopropyl(4-chlorophenyl)acetate

A 1 dram vial was charged with 1 ml of a toluene solution of 0.6 mmol of alpha-cyano-3-phenoxybenzyl alcohol prepared as in Embodiment 9 and having an isomer ratio of R/S = 72/28. To this was added 0.121 ml of (4-chlorophenyl)isopropylketene followed by 0.0084 ml of triethylamine. The reaction mixture became warm; reaction was complete in less than 1 minute. The mixture was washed with 1 N HCl, water, dried ($MgSO_4$), and diluted to 2 ml with toluene. Liquid chromatographic analysis on a chiral Pirkle column gave the following ratio of isomers: S-alpha-cyano-3-phenoxybenzyl R-isopropyl(4-chlorophenyl)acetate/R,S-isomer/S,S-isomer/R,R-isomer = 13.6/42.2/30.6/13.6.

Embodiments 112-121 - S-alpha-Cyano-3-phenoxybenzyl Isopropyl(4-chlorophenyl)acetate

Following procedures similar to those described in Embodiments 110 and 111 above, equimolar amounts of nonchiral tertiary amines, (4-chlorophenyl)isopropylketene and S-alpha-cyano-3-phenoxybenzyl alcohol were reacted at 0° C.

The results of these experiments are set forth in Table I below, in which the capital letters A and B represent the S or the R absolute configuration, respectively, in the acid moiety of the ester product and α stands for the S absolute configuration in the alcohol moiety of the ester product.

Table 1

| Embodiment | Tertiary Amine | Yield, % | Selectivity, %, to |
|---|---|---|---|
| 112 | 1,4-diazabicyclo[2.2.2]octane | 60.8 Bα | Bα, 68.1 |
| 113 | 1-methylimidazole | 46.4 Aα | Aα, 52.0 |
| 114 | 2,6-lutidine | 63.7 Bα | Bα, 71.1 |
| 115 | dimethylbenzylamine | 53.8 Bα | Bα, 58.9 |
| 116 | dimethyldodecylamine | 57.6 Bα | Bα, 63.3 |
| 117 | imidazole | 42.3 Bα | Bα, 50.7 |
| 118 | tricapylamine | 54.2 Bα | Bα, 60.4 |
| 119 | pyridine | 58.9 Bα | Bα, 64.6 |
| 120 | pyridine (excess ketene) | 55.5 Bα | Bα, 61.3 |
| 121 | dimethylaniline | 44.9 Bα | Bα, 50.4 |

All reactions used aliquots of the same sample of alpha-cyano-3-phenoxybenzyl alcohol which was about 86% S-isomer.

Choice of different reaction conditions within the scope of the invention can alter the rate of reaction and amount of predominant isomer. Of course, substitution of pure optically-active R- or S-alpha-cyano-3-phenoxybenzyl alcohol or a more highly enriched mixture thereof in Embodiments 110-121 above is within the scope of the invention, and increases the amount of R or S isomer product, respectively.

## Claims

1. A process for the preparation of a cyanomethyl ester of an alpha-chiral carboxylic acid comprising treating a non-symmetrical ketene with an alpha-hydroxynitrile or with an aldehyde or ketone and a source of cyanide ions.

2. A process according to claim 1 conducted in the presence of an optically-active tertiary amine catalyst.

3. A process according to claim 2 wherein the ketene is treated with an optically-active alpha-hydroxynitrile.

4. A process according to claim 3 wherein the amine is an optically-active histidine-containing peptide catalyst.

5. A process according to claim 4 which is conducted in the presence of a non-hydroxylic solvent.

6. A process according to claim 5 wherein the non-symmetrical ketene is treated with an alpha-hydroxynitrile having the S-configuration.

7. A process according to claim 6 wherein the catalyst is histidine or a histidine-containing di- or polypeptide in which at least one of the histidyl-free N-H and free COOH groups is optionally modified with a protecting group into the form of an amide, or an acid addition salt thereof, and ester group respectively; or the reaction product of one mole of a histidine or histidine-containing di- or polypeptide with from about one mole of a ketene.

8. A process according to claim 7 wherein the histidine moiety in the catalyst is histidine, 3-methyl-histidine, 1-methyl-histidine, 1-ethyl-histidine, 1-propyl-histidine or 1-benzyl-histidine.

9. A process according to claim 8 wherein the catalyst is a cyclic dipeptide containing a histidine moiety and an alanine moiety.

10. A process according to claim 7 wherein the catalyst is optically active and selected from histidine, alpha-methyl-histidine, 1-methyl-histidine, 3-methylhistidine, cyclo(histidyl-histidine), (benzyloxycarbonylalanyl)histidine methyl ester, cyclo(alanyl-histidine), cyclo(beta-phenylalanyl-histidine), cyclo(beta-phenylalanyl-1-methyl-histidine), cyclo(beta-phenylalanyl-3-methyl-histidine), histidine methyl ester hydrochloride, histidine ethyl ester dihydrochloride, anserine, cyclo(valyl-histidine), glycyl-histidine, cyclo(phenylalanyl-glycyl-histidine), cyclo(leucyl-histidine), cyclo(homophenylalanyl-histidine), cyclo-(phenylalanyl-methylhistidine), N-alpha-(beta-naphthoyl)histidine, histidyl-alanine, histidyl-phenylalaninamide hydrochloride, histidyl-beta-phenylalanine, cyclo(histidyl-proline) or cyclo(glycyl-histidine) in free or protected form or a reaction product thereof with a ketene.

11. A process according to claim 10 wherein the alpha-hydroxynitrile is an S-alpha-cyano alcohol having the formula

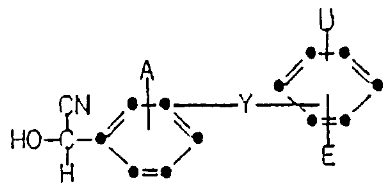

wherein Y is O; A, D and E each independently is a hydrogen atom, a halogen atom having an atomic number of from 9 to 35, inclusive, or an alkyl or alkoxy group containing 1 to 6 carbon atoms, each optionally substituted by one or more halogen atoms having an atomic number of from 9 to 35, inclusive.

12. A process according to claim 11 wherein S-alpha-cyano alcohol is S-alpha-cyano-3-phenoxybenzyl alcohol.

13. A process according to claim 11 wherein the S-alpha-cyano alcohol is S-alpha-cyano-4-fluoro-3-phenoxybenzyl alcohol or S-alpha-cyano-3-(4-fluorophenoxy)benzyl alcohol.

14. A process according to claim 7 wherein the optically-active ester product is S-alpha-cyano-3-phenoxybenzyl S-alpha-isopropyl-4-chlorophenylacetate or a mixture enriched therein.

15. A process according to claim 1 wherein the non-symmetrical ketene has the formula

$$R^2-\overset{R^1}{\overset{|}{C}} = C=O$$

wherein $R^1$ is isopropyl or cyclopropyl; $R^2$ is different from $R^1$ and is an alkyl group containing 1 to 6 carbon atoms; an alkenyl group containing 2 to 6 carbon atoms; a naphthyl group, a phenyl group or a (benzyloxycarbonyl)phenylamino group each optionally ring substituted by one or more of halogen, alkyl, haloalkyl, alkoxy, haloalkoxy in which the halogens are bromine, chlorine or fluorine and the alkyl group contains 1 or 4 carbon atoms.

16. A process according to claim 7 wherein the non-symmetrical ketene $R^1$ is isopropyl; $R^2$ is a phenyl group para-substituted by halogen, alkyl or haloalkoxy in which the alkyl contain 1 to 4 carbon atoms.

17. A process according to claim 9 wherein the non-symmetrical ketene is (4-chlorophenyl)-isopropylketene.

18. A process according to claim 1 wherein the ketene is prepared by treating an acid halide with a tertiary amine.

19. A process according to claim 1 wherein the optically active alpha-hydroxynitrile is prepared by treating the corresponding aldehyde or ketone with a source of cyanide ions in a substantially water-immiscible, aprotic solvent and in the presence of a cyclo(D-phenyl-alanyl-D-histidine) dipeptide catalyst.